# EUROPEAN PATENT SPECIFICATION

(11) **EP 3 229 719 B1**
(45) Date of publication and mention of the grant of the patent: **17.07.2019**
(21) Application number: 15831098.7
(22) Date of filing: 10.12.2015
(51) Int. Cl.: A61B 18/26, A61B 18/24, A61B 18/20, A61B 17/22, A61N 5/06, A61B 1/018, A61B 1/307, A61N 5/067, A61B 18/00

(54) **MEDICAL DEVICE**
MEDIZINISCHE VORRICHTUNG
DISPOSITIF MÉDICAL

(30) Priority: 11.12.2014 US 201462090732 P
(43) Date of publication of application: 18.10.2017
(73) Proprietor: Boston Scientific Scimed, Inc., Maple Grove, MN 55311 (US)
(72) Inventor: CIULLA, Ronald, Westford, MA 01886 (US); HERA, Mark, Holden, MA 01520 (US); GODDARD, James, Pepperell, MA 01463 (US)
(74) Representative: Pfenning, Meinig & Partner mbB
(86) International application number: PCT/US2015/064892
(87) International publication number: WO 2016/094610

(56) References cited:
- WO-A1-91/06271
- WO-A1-99/22656
- WO-A2-00/48525
- US-A- 5 487 740

## Description

### Technical Field

The present disclosure relates generally to medical devices. More particularly, the disclosure relates to medical devices for use in medical applications, such as, for example, breaking objects into smaller particles, and removing the resulting particles from a patient. The disclosure also relates to methods of using such instruments.

### Background of the Disclosure

The incidence of hospitalization for the removal of urinary calculi, commonly referred to as kidney stones, has been estimated at 200,000 cases per year. A vast majority of these patients pass their stones spontaneously; however, in the remainder, the kidney stone(s) become impacted in the ureter, a muscle tube joining the kidney to the bladder, or the stone may be too large to pass spontaneously. An impacted kidney stone is a source of intense pain and bleeding, a source of infection and, if a stone completely blocks the flow of urine for any extended length of time, can cause the loss of a kidney.

A device for removal of urinary calculi, using a laser is disclosed for example in WO 00/48525.

Recently, various methods have been utilized to break the stone into smaller fragments. One such method is stone dusting. Stone dusting is used by some urologists to fragment and evacuate stones from a kidney and is often performed by a ureteroscope. Intense light energy from a laser is passed through a fiber within the ureteroscope to break the stone into increasingly smaller pieces. Rather than breaking up the stone into chunks, which are removed by baskets, dusting generates very small fragments that are capable of being passed naturally. However, in some cases, these small stone fragments may not pass naturally. For example, the stone fragments may collect in an area of the kidney where they are less likely to flow out naturally, such as the lower pole of the kidney. In theory, any of these small stone fragments that do not evacuate through natural urine flow, could be a seed for new stone growth. Additionally, in some cases, the stone and/or the stone fragments may be pushed away from the ureteroscope by the laser, thus making it impossible to continue to break the stone or stone fragments into smaller fragments without repositioning the ureteroscope. The disclosure addresses the above-mentioned process and other problems in the art.

### SUMMARY OF THE DISCLOSURE

The invention is defined in the claims, other embodiments being merely exemplary.

It is to be understood that both the foregoing general description and the following detailed description are exemplary and explanatory only and are not restrictive.

In one example, a medical device may include a tube having a distal end, a proximal end, at least one side port located at the distal end, and a distal end face; a lumen in communication with the at least one side port and fluidly connecting the proximal end of the tube with the at least one side port; and a working channel extending from the proximal end to the distal end face of the tube.

Examples of the medical device may additionally and/or alternatively include one or more other features. Features of the various examples described in the following may be combined unless explicitly stated to the contrary. For example, a laser disposed within the working channel. In another example, the working channel includes a first opening located proximal to the distal end face of the tube and a second opening located proximal to the distal end face of the tube. A laser may extend from a laser control through the first opening in the working channel and to the distal face end. A vacuum source may be connected to the second opening in the working channel. A vacuum source may be connected to the working channel. A fluid supply assembly may be connected to the lumen. A second side port may be in fluid communication with a second lumen. The at least one side port may be angled. An illumination device may be located at the distal end face. Additional or alternatively, an imaging device may be located at the distal end face.

In another example, a method of operating a medical device may include coupling a vacuum source to a working channel of the medical device; and coupling a fluid supply assembly to a lumen having at least one side port located at a distal end of the medical device.

Examples of the method of operating the medical device may additionally and/or alternatively include one or more other features. For example, coupling a laser through the working channel. The laser and the vacuum source may be coupled to the working channel at the same time. The coupling of the vacuum source and the fluid supply assembly may further include coupling a flow rate controllable vacuum source and a fluid supply assembly, respectively.

In another example, a method of operating a medical device may include positioning a medical device at a target area, the medical device including a working channel and a lumen, wherein the lumen is in fluid communication with at least one side port; supplying fluid through the lumen to the at least one side port; applying suction through the working channel; disposing a laser within the working channel; and initiating the laser.

Examples of the method of operating the medical device may additionally and/or alternatively include one or more other features. For example, the step of applying suction may be performed after the step of initiating the laser; removing the laser before applying suction. The at least one side port may be angled so as to direct the introduced fluid distally. Additionally or alternatively, a flow rate through the lumen may match the flow rate through the working channel.

### BRIEF DESCRIPTION OF THE DRAWINGS

The accompanying drawings, which are incorporated in and constitute a part of this specification, illustrate various examples and together with the description, serve to explain the principles of the disclosure.
FIG. 1 illustrates an exemplary ureteroscope, including a tube, a handle portion, a fluid supply assembly, a laser source, an illumination source, an imaging apparatus, and a vacuum source;
FIG. 2 illustrates an exemplary distal end of the ureteroscope of FIG. 1; and
FIG. 3 is a block diagram of an exemplary method of using the ureteroscope disclosed herein.

### DETAILED DESCRIPTION

Reference will now be made in detail to aspects of the disclosure, examples of which are illustrated in the accompanying drawings. Wherever possible, the same reference numbers will be used throughout the drawings to refer to the same or like parts.

### Overview

Aspects of the present disclosure relate to systems and methods for breaking kidney stones into smaller particles and removing those particles from the body. The medical device described herein may work by positioning within a body, a ureteroscope and a laser disposed within a lumen of the ureteroscope. The laser may be used to break up kidney stones into particles. During the laser process or after removal of the laser from the body and/or lumen, the ureteroscope may vacuum the resulting particles from the body. More specifically, in some examples, the ureteroscope includes a tube with at least two lumens. A first lumen may provide fluid to irrigate a target area. A second lumen may be a working channel, configured to receive and position a laser and to provide suction to carry out the irrigation fluid along with the particles.

The target area may be any location. In some examples, the target area may be anywhere within a urinary tract including, but not limited to, a kidney. In some examples, the target area may be a site in the body where a kidney stone(s) is known or suspected to be located.

### Detailed Examples

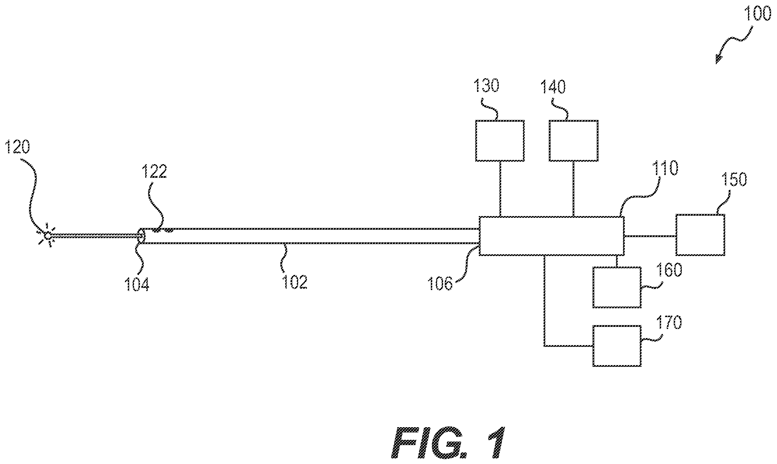

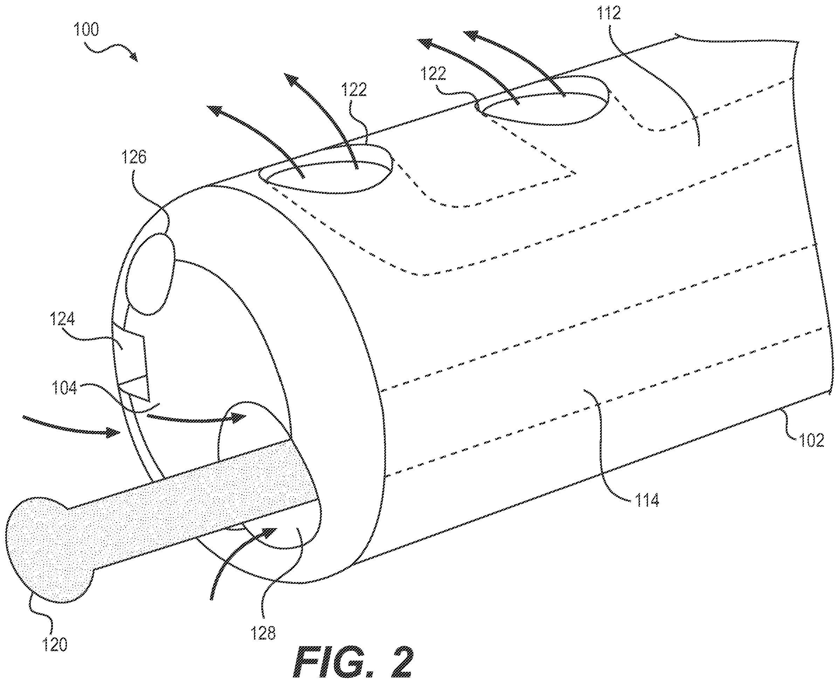

FIG. 1 illustrates an exemplary medical device 100 for removing stone fragments/dust. The device 100 may include a tube 102. Tube 102 may be a hollow, flexible, elongate tube having side ports 122, a distal end 104, a proximal end 106, and independent first and second lumens, irrigation lumen 112 and working channel 114 (FIG. 2). Irrigation lumen 112 and working channel 114 may extend between distal end 104 and proximal end 106 of tube 102. Additionally or alternatively, irrigation lumen 112 and working channel 114 may terminate proximal to distal end 104 of tube 102. For example, in some examples, irrigation lumen 112 may terminate at side ports 122 of tube 102, wherein side ports 122 may be open to allow flow of irrigation fluid. Working channel 114 may remain open at the distal end 104 of tube 102 to allow introduction of a laser 120 and/or application of suction. Proximal end 106 of tube 102 may be coupled to a handle portion 110. The handle portion 110 and/or the proximal end 106 of tube 102 may be attached to a laser control 130, a fluid supply assembly 140, a vacuum source 150, an illumination source 160, and/or an imaging apparatus 170.

### A. The Handle Portion

Handle portion 110 can be attached to tube 102 by, for example, welding, a locking configuration adhesive, or integrally formed with tube 102. The handle portion 110 may include a plurality of ports. For example, a first port may place irrigation lumen 112 in fluid communication with a fluid supply assembly 140 and a second port may place working channel 114 in fluid communication a vacuum source 150, respectively. Additional ports and lumens may be provided for supplying and/or controlling a laser, illumination device, and/or an imaging device located at or near distal end 104 of tube 102. For example, working channel 114 may include two ports, a first for connecting the vacuum source 150 and a second for connecting laser 120 and/or laser source 130. The handle portion 110 may include an actuating mechanism (not shown) to actuate one or more medical devices that may be located at the distal end 104 of tube 102. For example, the handle portion may include an actuating mechanism to power on or off the laser, the illumination device and/or the imaging device.

### B. The Tube

Tube 102 may be circular, ovoidal, irregular, and/or any shape suitable to enter a body. Further, tube 102 may have a uniform shape along its length, or may having a varying shape, such as a taper at the distal end to facilitate insertion within the body. Depending upon the particular implementation and intended use, the length of tube 102 may vary. The diameter of tube 102 may be tailored based on the body cavity. Similarly, depending upon the particular implementation and intended use, tube 102 can be rigid along its entire length, flexible along a portion of its length, or configured for flexure at only certain specified locations.

In one example, tube 102 may be flexible, adapted for flexible steering within bodily lumens, as understood in the art. For example, tube 102 can include a steering system (not shown) to move at least a portion (e.g., distal end 104) up/down and/or side-to-side. Additional degrees of freedom, provided for example via rotation, translational movement of tube 102, or additional articulation of bending sections, may also be implemented. Examples of such steering systems may include at least one of or all of pulleys, control wires, gearing, and electrical actuators.

Tube 102 may be formed of any suitable material having sufficient flexibility to traverse body cavities and tracts. In general, tube 102 may be made of any suitable material that is compatible with living tissue or a living system. That is, the tube 102 may be non-toxic or non-injurious, and it should not cause immunological reaction or rejection. In some examples, tube 102 may be made of polymetric elastomers, rubber tubing, and/or medically approved polyvinylchloride tubing. Polymeric elastomers may be, for example, EVA (Ethylene vinyl acetate), silicone, polyurethane, and/or C-Flex.

Tube 102 may be designed to impose minimum risk to the surrounding tissues while in use. To this end, one or more portions of tube 102 may include atraumatic geometrical structures, such as rounded or beveled terminal ends or faces, to reduce trauma and irritation to surrounding tissues.

To effectively maneuver the tube 102 within a body cavity, the operator may need to know the exact location of the tube 102 in the body cavity at all times. To this end, one or more portions of the tube 102 may be radiopaque, such as by inclusion of barium sulfate in plastic material or inclusion of one or more metal portions, which provide sufficient radiopacity. Additionally or alternatively, distal end 104 of tube 102 may include radiopaque or sonoreflective markers (not shown). These markings facilitate detection of a position and/or orientation of the tube 102 within a patient's body, and an operator, with the aid of suitable imaging equipment, may track the path followed by tube 102. This may help the operator avoid potential damage to sensitive tissues. By using fluoroscopic guidance, the space within tube 102 that would be needed for direct visualization (e.g., an imaging apparatus) may instead be used to maximize the size of the lumens and/or the flow rate of introduced fluid.

Further, the tube 102 may include any suitable coating and/or covering. For example, the outer surface may include a layer of lubricous material to facilitate insertion through a body lumen or surgical insertion. Further, tube 102 may be coated with a biocompatible material such as Teflon. To inhibit bacterial growth in the body cavity, tube 102 may be coated with an antibacterial coating. Further, an anti-inflammatory substance may also be applied to the outer surface of the tube 102, if required.

FIG. 2 illustrates an exemplary distal end 104 of ureteroscope 100. In the example shown in FIG. 2, irrigation lumen 112 has two laterally facing side ports 122 and working channel 114 has one distal facing opening 128.

Irrigation lumen 112 may be in fluid communication with fluid supply assembly 140 and side ports 122 may provide for introduction of fluid into a desired site, such as the kidney. An operator may establish the connection between irrigation lumen 112 and the fluid supply assembly 140. The fluid supply assembly 140 may provide fluid, through irrigation lumen 112, to the distal end 104 of tube 102 and into a desired site, such as the kidney. The fluid supply assembly 140 may be any device and/or devices that can supply fluid to irrigation lumen 112. The fluid supply assembly 140 may include, but is not limited to, a fluid source, a pump, a control system, a heat exchanger, a filter, a temperature sensor, a pressure sensor, a supply line, and/or various user input devices. In some examples, the fluid supplied is a saline solution, for example, 0.9% saline.

Irrigation lumen 112 may be any shape, including, but not limited to, circular, semi-circular, and non-circular. For example, irrigation lumen 112 may be a flattened tube. Such a shape may more effectively use the space with the ureteroscope. For example, such a shape may allow working channel 114 to have a larger cross-sectional area.

Side ports 122 may introduce fluid radially outward of tube 102 (e.g., approximately perpendicular to the longitudinal axis of tube 102) or side ports 122 may be angled toward the distal end of the ureteroscope so that the irrigation fluid is introduced toward the distal end of the ureteroscope. The angling of fluid introduced through side ports 122 may also include angling the passage(s) connected to side ports 122. The angle of the introduction of fluid may be greater than approximately 10 degrees from longitudinal axis of tube 102 to less than approximately 90 degrees from the longitudinal axis of tube 102, preferably between approximately 20 degrees and approximately 80 degrees. In some examples, the preferred angle of introduction of fluid may be between approximately 30 degrees and approximately 60 degrees. Angling the introduction of fluid toward the distal end of tube 102 may assist in pushing stone fragments/dust toward the vacuum, e.g., distal opening 128 of working channel 114. It should be noted that there may be any number of side ports 122, spaced any distance apart, and located anywhere along the radial surface of tube 102. While FIG. 2 illustrates a singular irrigation lumen in communication with side ports 122, each side port may be independent and in communication with a separate lumen.

By positioning side ports 122 on the sides of tube 102, more surface area will be available on the distal face of tube 102 and thus, the cross-sectional area of distal opening 128 of working channel 114 may be maximized.

Working channel 114 may be in fluid communication with at least one of or all of vacuum source 150, laser control 130, and distal opening 128. Distal opening 128 may be substantially perpendicular to the tube (e.g., as shown in FIG. 2), may be tapered or angled, or may be in any other suitable orientation. Distal opening may be any size and/or shape. The proximal end of working channel 114 may have any shape or configuration. For example, working channel 114 may have two openings or may fork at or near the proximal end 106 of tube 102. Working channel 114 may be configured in any way that would allow for working channel 114 to be simultaneously connected to vacuum source 150 and laser control 130. This may allow laser 120 to be disposed within working channel 114 at the same time suction is being applied through working channel 114.

An operator may connect working channel 114 to vacuum source 150 (e.g., house vacuum, vacuum pump, etc.). In some examples, working channel 114 and vacuum source 150 may be connected via a conduit. The conduit may be made of a flexible material (for example, a polymeric tube), a rigid material, or a combination of both flexible and rigid materials. In some examples, the conduit may be braided or wound with plastic or metal fibers to improve conduit's resistance against kink-formation or against collapse under vacuum pressure. In some examples, the conduit may include coatings on its inside or outside surface for various purposes, for example, for protection against corrosion and/or by body fluids. In general, the conduit may have any dimension suitable for its intended use. In some examples, an elongated polymeric or polypropylene tube may serve as the conduit.

Laser 120 may be introduced into a patient through working channel 114. Laser 120 may be connected to and/or controlled by laser control 130. Laser 120 may be utilized to break up kidney stones into smaller stone fragments.

In addition to separately and/or simultaneously connecting vacuum source 150 and laser control 130 to working channel 114, working channel 114 may be for connection to or the positioning of other instruments. For example, irrigation fluid may also be injected through working channel 114.

As shown in FIG. 2, in some examples, irrigation lumen 112 may open to the body cavity through side ports 122 and working channel 114 may open to the body cavity through the distal end 104 of tube 102. This configuration may improve the ability to break kidney stones by creating an antiretropulsion effect. By applying suction through working channel 114, a kidney stone may be pulled toward laser 120, thus countering the effect of the laser energy pushing the kidney stone away. This configuration thus assists in generating the smaller stone fragments by pulling the stones into the reach of laser 120. It may also improve the suction of the resulting stone fragments into working channel 114 and out of the body. By having irrigation from the side, the inflow fluid is less likely to interfere with the vacuuming of the stone dust. For example, this configuration may provide the desired effect of maintaining a pressure equilibrium through introduction of fluid, but avoid the undesired effect of fluid introduction pushing kidney stones and/or stone fragments/dust away from the distal opening 128 of working channel 114. Additionally or alternatively, this configuration may push stone fragments/dust that are located proximal to the distal end 104 of tube 102 to a position distal to the distal opening 128 of working channel 114, thus allowing stone fragments/dust to be suctioned out of the body into working channel 114.

While two lumens are illustrated in FIG. 2, tube 102 may include any number of lumens. The lumens included in tube 102 may be any size, shape, and/or in any configuration. Any lumen may include any suitable coating. For example, a lumen may include a layer of lubricous material to facilitate insertion of any instrument and/or device. In some examples, working channel 114 may be coated with a lubricous material to facilitate insertion of laser 120.

In some examples, the distal end 104 of tube 102 may include visualization devices such as imaging device 124 and/or an illumination device 126. These devices may be connected to imaging apparatus 170 and illumination source 160, respectively. As shown in FIG. 2, imaging device 124 and illumination device 126 may be disposed on a distal facing surface of tube 102. These devices may be integrally formed with the tube 102 or may attach to the distal end 104 using known coupling mechanisms. Alternatively, the visualization devices may be detachably introduced into tube 102 through irrigation lumen 112 and/or working channel 114 when required. For example, the proximal end of irrigation lumen 112 and/or working channel 114 may be forked to allow introduction of additional devices as well as a connection to either fluid supply apparatus 140 and/or vacuum source 150. Additionally or alternatively, irrigation lumen 112 and/or working channel 114 may include side port(s) at proximal end 106 for introduction of additional devices.

### C. Exemplary Method of Operation

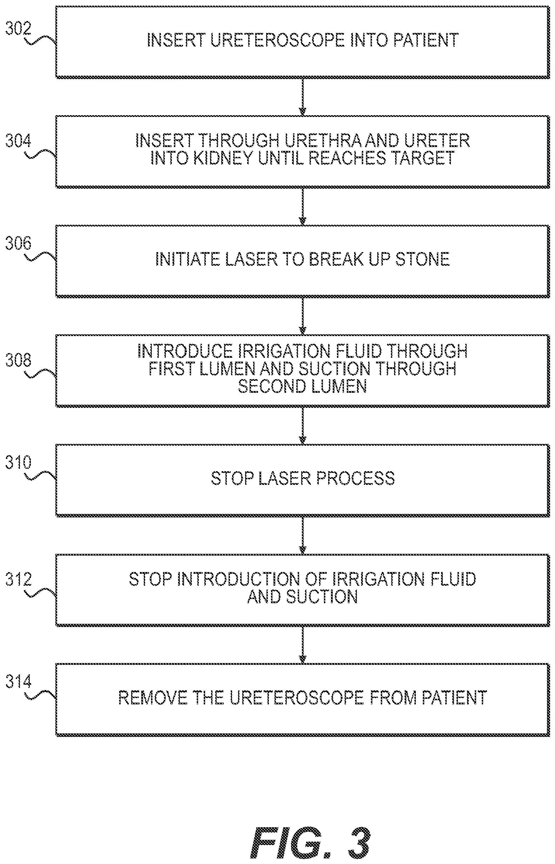

FIG. 3 illustrates an exemplary method of use of a medical device for breaking a kidney stone into stone fragments/dust and removing these stone fragments/dust from the body. For purposes of discussion, method 300 will be described using medical device 100 of FIG. 1 (including lumens 112 and 114), as described above, but method 300 is not intended to be limited thereto. As shown in FIG. 3, method 300 includes steps 302, 304, 306, 308, 310, 312, and 314. However, it should be noted that method 300 may include more or fewer steps as desired for a particular implementation and the order of the steps may be varied.

Method 300 may commence when an operator (e.g., a doctor or other medical personnel) inserts a ureteroscope, (ureteroscope 100, for example) into a patient (step 302). In step 304, an operator may insert the distal end 104 of the tube 102 into the patient's urethra. The operator may advance the tube 102 so that the distal end 104 passes into and through the urinary bladder, into and through the ureter, and into the kidney. The operator may position the distal end 104 of the tube 102 within the patient's kidney. The operator may position a distal facing surface of tube 102 and/or distal opening 128 of working channel 114 proximate a target area. A target area may a site where stones and/or stone fragments are known or suspected to be located. An imaging device may be utilized to determine the location of stone(s), as known in the art.

The ureteroscope may be adjusted so that a laser may be aimed at the located stone(s). For example, laser 120 may be disposed within working channel 114 and distal opening 128 of working channel 114 may be positioned proximate to the stone. In some examples, a laser is disposed within the ureteroscope during step 304. In other examples, the laser may be only partially disposed within the ureteroscope or may be external to the ureteroscope during step 304. The laser may then extend through the ureteroscope 100 to distal end 104 once the ureteroscope 100 is in the operator's desired position.

Once the laser is in a sufficient position to aim for the kidney stone, the operator may initiate the laser to break up the kidney stone (step 306). Method 300 may then proceed to step 308. In step 308, the operator may turn on the fluid supply assembly 140 to introduce fluid through irrigation lumen 112 to the target area and/or the operator may turn on the suction to pull the stone fragments/dust into working channel 114. The fluid may be introduced in a continuous flow. In some examples, the fluid may be initially introduced in a pulsed flow. A pulsed flow may dislodge stone fragments/dust adhering to various surfaces within the patient's body. The pulsed flow may create turbulence. The turbulence may stir up any stone fragments/dust which may be located in an area where the stone fragments/dust may be less likely to flow out naturally (e.g. the lower pole of the kidney). The turbulence may aid in keeping stone fragments/dust in suspension so that they may be more effectively suctioned out through working channel 114.

The fluid may be provided to the supply irrigation lumen 112 at a variety of flow rates. In some examples, the flow rate may be pulsed at a regular interval, e.g., every few minutes. The pulsed flow may be a flow that is either intermittently interrupted or simply reduced in rate on an intermittent basis. The flow rate may be pulsed at complex flow rate patterns such as periodic and aperiodic oscillatory patterns. For example, a pulse interval (time between pulse cycles) may be adjustable and may range e.g. from 100 pulses per second to 1 pulse cycle every 2 seconds. A pulse pattern may be pre-set, determined in real-time by the operator, or may be actively controlled and optimized based on parameters collected from sensing mechanisms and implemented by a processor. A pulsed flow may be created in any way. In one example, the pulse flow may be created by a mechanical pump. The mechanical pump may apply and release pressure on the fluid at intervals. The mechanical pump may be operated and controlled manually or a processor may control the pattern, duration, intensity, intervals, etc. of the pulses.

The suction may be applied at a flow rate that matches the flow rate of the introduced fluid. A matched flow rate may be any flow rate that prevents harm to the patient. For example, a matched flow rate may be any flow rate of the introduction of fluid in relationship to the flow rate of the suction that prevents the kidney from collapsing due to no fluid in the system, as known in the art. The matched flow rate may assist in maintaining a pressure equilibrium during operation of the device. In some examples, a pressure sensor may also be located at or near the target area and/or distal end 104 to assist in maintaining a pressure equilibrium.

The vacuum source 150 may allow the operator to vary the suction. The vacuum source 150 may be located near the patient or may be located remotely (such as a vacuum source located on a wall).

Once the operator determines a kidney stone has been broken into sufficiently small fragments or does not want to continue for other reasons, method 300 may proceed to step 310 and the laser process may be stopped. Once the stone fragments/dust have been sufficiently removed from the body, method 300 may proceed to step 312. In step 312, the operator may stop introduction of irrigational fluid and/or stop suction.

In some examples, the vacuum source does not operate at the same time as the laser. For example, step 310 may proceed step 308. In some examples, the laser is removed from working channel 114 before method 300 proceeds to step 308. It should be noted that the steps of method 300 may be performed in any order. For example, step 312 may proceed step 310 or steps 306 and 308 may be initiated concurrently, or step 308 may proceed step 306.

At any point, an operator may additionally choose to move the device within the patient. For example, an operator may choose to move the distal end 104 of tube 102 to the site of an additional kidney stones and/or lower into the kidney or to a location in which additional stone fragments/dust have accumulated. The purpose of repositioning the distal end 104 may be to reach stones or stone fragments that need to be broken into smaller pieces and/or reach stone fragments/dust that the device was previously unable to suction out of the body and into working channel 114. For example, some stone fragments/dust may be positioned proximally to the distal opening or positioned too distally to be captured by applied suction. An operator may reposition the distal end 104 of tube 102 any number of times. Once repositioned, any or all of steps 306-312 may be repeated at the new location.

Once an operator determines no more kidney stones and/or stone fragments/dust can and/or should be removed, method 300 may proceed to step 314 and the ureteroscope may be removed from the patient's body.

The many features of the disclosure are apparent from the detailed specification, and thus, it is intended by the appended claims to cover all such features of the disclosure which fall within the true spirit and scope of the disclosure. Further, since numerous modifications and variations will readily occur to those skilled in the art, it is not desired to limit the disclosure to the exact construction and operation illustrated and described, and accordingly, all suitable modifications and equivalents may be resorted to, falling within the scope of the disclosure.

## Claims

1. A medical device (100), comprising:
a tube (102) having a distal end (104), a proximal end (106), at least one side port (122) located at the distal end (104), and a distal end face;
an irrigation lumen (112) in communication with the at least one side port (122) and fluidly connecting the proximal end (106) of the tube (102) with the at least one side port (122);
a working channel (114) extending from the proximal end (106) to the distal end face of the tube (102), wherein the working channel (114) is configured to receive a laser (120) within the working channel (114) and to provide suction through a distal opening (128) of the working channel (114); and
a laser (120) disposed within the working channel (114).

2. The medical device (100) of claim 1, wherein the laser (120) is adapted to extend distally through the distal opening (128) of the working channel (114).

3. The medical device (100) of any of the preceding claims, wherein the working channel (114) includes a first opening located proximal to the distal end face of the tube (102) and a second opening located proximal to the distal end face of the tube (102).

4. The medical device (100) of claim 3, wherein a laser (120) extends from a laser control (130) through the first opening in the working channel (114) and to the distal face end.

5. The medical device (100) of any of claims 3 and 4, wherein a vacuum source (150) is connected to the second opening in the working channel (114).

6. The medical device (100) of any of claims 1-4, further comprising:
a vacuum source (150) connected to the working channel (114).

7. The medical device (100) of any of the preceding claims, further comprising:
a fluid supply assembly (140) connected to the lumen (112).

8. The medical device (100) of any of the preceding claims, further comprising:
a second side port in fluid communication with a second lumen.

9. The medical device (100) of any of the preceding claims, wherein the at least one side port (122) is angled toward the distal end (104) of the tube (102).

10. The medical device (100) of any of the preceding claims, further comprising:
an illumination device (126) located at the distal end face.

11. The medical device (100) of any of the preceding claims, further comprising:
an imaging device (124) located at the distal end face.

12. A method of operating a medical device (100), comprising:
coupling a vacuum source (150) to a working channel (114) of the medical device (100), wherein the medical device (100) is the medical device (100) of any one of the preceding claims; and
coupling a fluid supply assembly (140) to a lumen (112) having at least one side port (122) located at a distal end (104) of the medical device (100).

13. The method of claim 12, further comprising:
coupling a laser (120) through the working channel (114).

14. The method of 13, wherein the laser (120) and the vacuum source (150) are coupled to the working channel (114) at the same time.

15. The method of any of claims 12-14, wherein the coupling of the vacuum source (150) and the fluid supply assembly (140) further includes coupling a flow rate controllable vacuum source (150) and a fluid supply assembly (140), respectively.

## Patentansprüche

1. Medizinische Vorrichtung (100), welche aufweist:
ein Rohr (102) mit einem distalen Ende (104), einem proximalen Ende (106), zumindest einer Seitenöffnung (122), die sich an dem distalen Ende (104) befindet, und einer distalen Endfläche;
ein Spülungslumen (112) in Verbindung mit der zumindest einen Seitenöffnung (122), das fluidmäßig das proximale Ende (106) des Rohrs (102) mit der zumindest einen Seitenöffnung (122) verbindet;
einen Arbeitskanal (114), der sich von dem proximalen Ende (106) zu der distalen Endfläche des Rohrs (102) erstreckt, wobei der Arbeitskanal (114) konfiguriert ist zum Aufnehmen eines Lasers (120) innerhalb des Arbeitskanals (114) und zum Bereitstellen eines Ansaugens durch eine distale Öffnung (128) des Arbeitskanals (114); und
einen Laser (120), der innerhalb des Arbeitskanals (114) angeordnet ist.

2. Medizinische Vorrichtung (100) nach Anspruch 1, bei der der Laser (120) ausgestaltet ist, sich distal durch die distale Öffnung (128) des Arbeitskanals (114) zu erstrecken.

3. Medizinische Vorrichtung (100) nach einem der vorhergehenden Ansprüche, bei der der Arbeitskanal (114) eine erste Öffnung, die sich proximal von der distalen Endfläche des Rohrs (102) befindet, und eine zweite Öffnung, die sich proximal von der distalen Endfläche des Rohrs (102) befindet, enthält.

4. Medizinische Vorrichtung (100) nach Anspruch 3, bei der ein Laser (120) sich von einer Lasersteuerung (130) durch die erste Öffnung in dem Arbeitskanal (114) und zu der distalen Endfläche erstreckt.

5. Medizinische Vorrichtung (100) nach einem der Ansprüche 3 und 4, bei der eine Vakuumquelle (150) mit der zweiten Öffnung in dem Arbeitskanal (114) verbunden ist.

6. Medizinische Vorrichtung (100) nach einem der Ansprüche 1-4, welche weiterhin aufweist:
eine Vakuumquelle (150), die mit dem Arbeitskanal (114) verbunden ist.

7. Medizinische Vorrichtung (100) nach einem der vorhergehenden Ansprüche, welche weiterhin aufweist:
eine mit dem Lumen (112) verbundene Fluidzuführungsanordnung (140).

8. Medizinische Vorrichtung (100) nach einem der vorhergehenden Ansprüche, welche weiterhin aufweist:
eine zweite Seitenöffnung in Fluidverbindung mit einem zweiten Lumen.

9. Medizinische Vorrichtung (100) nach einem der vorhergehenden Ansprüche, bei der die zumindest eine Seitenöffnung (122) zu dem distalen Ende (104) des Rohrs (102) hin abgewinkelt ist.

10. Medizinische Vorrichtung (100) nach einem der vorhergehenden Ansprüche, welche weiterhin aufweist:
eine Beleuchtungsvorrichtung (126), die sich an der distalen Endfläche befindet.

11. Medizinische Vorrichtung (100) nach einem der vorhergehenden Ansprüche, welche weiterhin aufweist:
eine Abbildungsvorrichtung (124), die sich an der distalen Endfläche befindet.

12. Verfahren zum Betätigen einer medizinischen Vorrichtung (100), welches aufweist:
Koppeln einer Vakuumquelle (150) mit einem Arbeitskanal (114) der medizinischen Vorrichtung (100), wobei die medizinische Vorrichtung (100) die medizinische Vorrichtung (100) nach einem der vorhergehenden Ansprüche ist; und
Koppeln einer Fluidzuführungsanordnung (140) mit einem Lumen (112), das zumindest eine Seitenöffnung (122) hat, die sich an einem distalen Ende (104) der medizinischen Vorrichtung (100) befindet.

13. Verfahren nach Anspruch 12, welches weiterhin aufweist:
Koppeln eines Lasers (120) durch den Arbeitskanal (114).

14. Verfahren nach Anspruch 13, bei dem der Laser (120) und die Vakuumquelle (150) gleichzeitig mit dem Arbeitskanal (114) gekoppelt werden.

15. Verfahren nach einem der Ansprüche 12-14, bei dem das Koppeln der Vakuumquelle (150) und der Fluidzuführungsanordnung (140) weiterhin das Koppeln einer steuerbaren Strömungsraten-Vakuumquelle (150) beziehungsweise einer Fluidzuführungsanordnung (140) enthält.

## Revendications

1. Dispositif médical (100), comprenant :
un tube (102) qui comporte une extrémité distale (104), une extrémité proximale (106), au moins un orifice latéral (122) qui est localisé au niveau de l'extrémité distale (104) et une face d'extrémité distale ;
une lumière d'irrigation (112) qui est en communication avec l'au moins un orifice latéral (122) et qui connecte en termes de fluide l'extrémité proximale (106) du tube (102) avec l'au moins un orifice latéral (122) ;
un canal de travail (114) qui s'étend depuis l'extrémité proximale (106) jusqu'à la face d'extrémité distale du tube (102), dans lequel le canal de travail (114) est configuré de manière à ce qu'il reçoive un laser (120) à l'intérieur du canal de travail (114) et de manière à ce qu'il assure une aspiration au travers d'une ouverture distale (128) du canal de travail (114) ; et
un laser (120) qui est disposé à l'intérieur du canal de travail (114).

2. Dispositif médical (100) selon la revendication 1, dans lequel le laser (120) est adapté de manière à ce qu'il s'étende de façon distale au travers de l'ouverture distale (128) du canal de travail (114).

3. Dispositif médical (100) selon l'une quelconque des revendications précédentes, dans lequel le canal de travail (114) inclut une première ouverture qui est localisée de façon proximale par rapport à la face d'extrémité distale du tube (102) et une seconde ouverture qui est localisée de façon proximale par rapport à la face d'extrémité distale du tube (102).

4. Dispositif médical (100) selon la revendication 3, dans lequel un laser (120) s'étend depuis un moyen de commande/contrôle de laser (130) au travers de la première ouverture qui est ménagée dans le canal de travail (114) et jusqu'à la face d'extrémité distale.

5. Dispositif médical (100) selon l'une quelconque des revendications 3 et 4, dans lequel une source de vide (150) est connectée à la seconde ouverture qui est ménagée dans le canal de travail (114).

6. Dispositif médical (100) selon l'une quelconque des revendications 1 à 4, comprenant en outre :
une source de vide (150) qui est connectée au canal de travail (114).

7. Dispositif médical (100) selon l'une quelconque des revendications précédentes, comprenant en outre :
un assemblage d'alimentation en fluide (140) qui est connecté à la lumière (112).

8. Dispositif médical (100) selon l'une quelconque des revendications précédentes, comprenant en outre :
un second orifice latéral qui est en communication en termes de fluide avec une seconde lumière.

9. Dispositif médical (100) selon l'une quelconque des revendications précédentes, dans lequel l'au moins un orifice latéral (122) est angulé en direction de l'extrémité distale (104) du tube (102).

10. Dispositif médical (100) selon l'une quelconque des revendications précédentes, comprenant en outre :
un dispositif d'éclairage (126) qui est localisé au niveau de la face d'extrémité distale.

11. Dispositif médical (100) selon l'une quelconque des revendications précédentes, comprenant en outre :
un dispositif d'imagerie (124) qui est localisé au niveau de la face d'extrémité distale.

12. Procédé pour faire fonctionner un dispositif médical (100), comprenant :
le couplage d'une source de vide (150) sur un canal de travail (114) du dispositif médical (100), dans lequel le dispositif médical (100) est le dispositif médical (100) selon l'une quelconque des revendications précédentes ; et
le couplage d'un assemblage d'alimentation en fluide (140) sur une lumière (112) qui comporte au moins un orifice latéral (122) qui est localisé au niveau d'une extrémité distale (104) du dispositif médical (100).

13. Procédé selon la revendication 12, comprenant en outre :
le couplage d'un laser (120) par l'intermédiaire et au travers du canal de travail (114).

14. Procédé selon la revendication 13, dans lequel le laser (120) et la source de vide (150) sont couplés sur le canal de travail (114) en même temps.

15. Procédé selon l'une quelconque des revendications 12 à 14, dans lequel le couplage de la source de vide (150) et de l'assemblage d'alimentation en fluide (140) inclut en outre, de façon respective, le couplage d'une source de vide pouvant être commandée/contrôlée en termes de débit d'écoulement (150) et d'un assemblage d'alimentation en fluide (140).
